# EUROPEAN PATENT APPLICATION

(11) **EP 0 914 834 A2**
(43) Date of publication of application: **12.05.1999**
(21) Application number: 98402764.9
(22) Date of filing: 06.11.1998
(51) Int. Cl.: A61L 27/00, C08L 75/04, A61L 31/00, A61L 29/00, A61L 33/00

(54) **Anti-thrombogenic polyurethane-hydrophilic polymer blend**

(30) Priority: 06.11.1997 KR 9758495
(71) Applicant: KOREA RESEARCH INSTITUTE OF CHEMICAL TECHNOLOGY, Daejeon, 305-343 (KR)
(72) Inventor: Kim, Sung Soo, Yusung-ku, Daejeon 305-390 (KR); Shin, Byung Chul, Yusung-ku, Daejeon 305-333 (KR); Kim, Hyung Woo, Dukjin-ku, Junju, Junrabuk-do 561-160 (KR); Yoo, Young Mi, Chungju, Choongchungbuk-do, 361-280 (KR)
(74) Representative: Kédinger, Jean-Paul

(57) **Abstract**

The present invention relates to an anti-thrombogenic polyurethane-hydrophilic polymer blend and more particularly, to the anti-thrombogenic polyurethane-hydrophilic polymer blend having an excellent anti-thrombogenesis as an useful material for artificial organ, wherein the polyurethane-based resin employed as the blood-compatibility material is homogeneously mixed with the hydrophilic polymer such as polyvinyl alcohol and polyhydroxyethylmethyl methacrylate.

## Description

### BACKGROUND OF THE INVENTION

### Field of the Invention

The present invention relates to an anti-thrombogenic polyurethane-hydrophilic polymer blend and more particularly, to the anti-thrombogenic polyurethane-hydrophilic polymer blend having an excellent anti-thrombogenesis as an useful material for artificial cardiovascular organs, wherein the polyurethane-based resin employed as the blood-compatibile material is homogeneously mixed with the hydrophilic polymer such as polyvinyl alcohol and polyhydroxyethylmethyl methacrylate.

### Description of the Related Art

Generally, a blood-compatibile material (anti-thrombogenic material) has been used not only as artificial organs including artificial vessel, artificial cardiac valve and artificial heart, which is directly contacted to blood, but also as medical devices including a protective membrane of biosensor designed to measure a concentration of oxygen, pH of blood and so on, catheter inserted in blood vessel, etc.

The blood-compatibile material should not produce any toxicity, carcinogenesis, inflammation, thrombogenesis, erythrocytolysis and other adverse effects. For example, a small-radius artificial blood vessel using thrombogenic material may cause vascular occlusion in the human body. The unsuccessful development of artificial heart lies mainly in thrombogenesis associated with an artificial heart. The long-term use of biosensor has not been available due to a thrombus present in its protective membrane, which lowers its efficiency significantly. Under such circumstances, many researches have focused on development of some blood-compatibile materials having an excellent anti-thrombogenic property.

Polyurethane may be employed as a blood-compatibility material, since it has better anti-thrombogenic property and mechanical strength and its elasticity and flexibility are similar to those of blood vessel and heart. Nevertheless, polyurethane in an original form may not be applicable to any artificial organ such as small-radius artificial vessel, which requires excellent anti-thrombogenic property.

Hitherto, various methods of improving the anti-thrombogenic property of polyurethane resin have been disclosed as follows: a) a method of grafting polyethylene glycol with polyurethane [J. Biom. Mat. Res., 87(1989)]; b) a method of introducing sulfone group [U.S. Patent No. 4,882,148] and phosphoryl choline [Int. J. Artif. Organs, 499(1994)] to polyurethane; c)a method of introducing heparin to polyurethane [J. Biom. Mat. Res., 423(1989)]; and d) a method of adsorbing albumin into polyurethane [U.S. Patent No. 5,073,171].

These anti-thrombogenic materials prepared from the above-mentioned methods have failed to meet some artificial organs such as a small-radius artificial vessel, which requires excellent anti-thrombogenic property.

In addition, another methods of improving the anti-thrombogenic property of polyurethane resin have been introduced by using a surface modifier such as polyethylene oxide or anti-thrombogenic materials was introduced to urethane. However, these methods have also recognized some shortcomings in that a) necessary chemical reactions are impeding factors to easily prepare the anti-thrombogenic material, b) with the surface reactions only, any surface modifier or anti-thrombogenic material is not sufficiently introduced into the polymer owing to limitedness of the amount of reactive groups, and c) when the surface of polyurethane resin is contaminated or deteriorated, its anti-thrombogenic property becomes inactivated.

### SUMMERY OF THE INVENTION

To overcome the aforementioned shortcomings that the conventional methods have faced, the inventor et al. have completed this invention by manufacturing an anti-thrombogenic polyurethane-hydrophilic polymer blend fabricated in such a manner that a hydrophilic polymer such as polyvinyl alcohol is mixed with a conventionally used polyurethane-based resin as a anti-thrombogenic material. The polymer blend of this invention may effectively contribute to inhibition of thrombogenesis with less adhesion to platelet and less activation of platelet.

Therefore, an object of this invention is to provide an anti-thrombogenic polyurethane hydrophilic polymer blend useful for the manufacture of artificial organ (artificial vessel, etc.) and medical device, which require excellent anti-thrmbogenic property.

### Detailed Description of the Invention

The present invention is characterized by an anti-thrombogenic polyurethane-hydrophilic polymer blend, wherein the hydrophilic polymer, being solubilized or swelled in water, is homogeneously mixed with the polyurethane-based resin.

This invention is explained in more detail as set forth hereunder:

A polymer blend of this invention, so prepared from the mixture containing a hydrophilic polymer and the conventional polyurethane resin as a blood-compatibility material, is designed to improve its anti-thrombogenic property. The blend material shows a structure entangled with polyurethane-based polymer chain in order to ensure that any hydrophilic polymer chains are not dissolved in water in the same manner as do in the body. With such Structure, hydrated hydrophilic polymer chains at the surface of the polymer blend are infiltrated into the blood as a form of cilia; since these hydrated chains have a high fluidity, they may contribute much to inhibition of thrombogenesis in a manner to prevent adsorption of thromobogenic proteins (e.g., fibrinogen and immunologic protein) and adherence of platelet and thrombus.

In other words, the conventional methods have also recognized some shortcomings in that a) necessary chemical reactions are impeding factors to easily prepare the anti-thrombogenic material, b) with the surface reactions only, any surface modifier or anti-thrombogenic material is not sufficiently introduced into the polymer owing to limitedness of the amount of reactive groups, and c) when the surface of polyurethane resin is contaminated or deteriorated, its anti-thrombogenic property becomes inactivated.

Unlike the conventional methods, this invention has several advantages in that a) with the method of mixing hydrophilic polymer with polyurethane, the blood compatible material may be easily prepared and larger amount of hydrophilic polymer as a surface modifier may be introduced at the surface of the polyurethane resin than the conventional methods, and c) larger distribution of hydrophilic polymers at the surface may minimize a poor anti-thrombogenic property clue to surface contamination.

According to this invention, examples of hydrophilic polymer designed to improve the blood-compatible of polyurethane-based resin may include any hydrophilic polymer which is dissolved or swelled in water. It is in particular preferred to use the following materials such as polyvinyl alcohol, polyhydroxyethylmethyl methacrylate, polyacrylic acid, polyacryl amide, polyvinyl pyrrolidone and polyethylene oxide. Also, it is preferred that the hydrophilic polymer is used in the range of 30 - 70 weight % in proportion to 100 weight % of the polyurethane-based resin; if the amount is less than 30 weight %, the anti-thrombogenesis is not sufficiently accomplished, but in case of exceeding 70 weight %, the dissolved hydrophilic polymer comes out with its weak mechanical strength.

Further, according to this invention, both polyurethane and hydrophilic polymer are dissolved in the presence of an appropriate solvent for its better mixing to prepare a polymer blend solution. Hence, examples of solvent include some polar solvents such as dimethylsulfoxide, dimethylformamide, tetrahydrofuran, etc. In order to enhance the solubility, the reaction is carried out at the temperature of about 120 °C. The polymer blend solution, so prepared, may be applicable depending on the purposes of use.

As described above, the polyurethane-hydrophilic polymer blend of this invention may be employed in artificial organs and medical devices which serve to directly contact with blood, since it exhibits its excellent anti-thrombogenic property.

According to this invention, an artificial organ refers to artificial vessel, artificial kidney, artificial heart, artificial liver, artificial lung and artificial cardiac valve. In addition, a medical device also refers to catheter, sensor and stent.

The following specific examples are intended to be illustrative of the invention and should not be construed as limiting the scope of the invention as defined by appended claims.

### Example 1

10g of polyurethane and 10g of polyvinyl alcohol (polymerization degree: 1,500) were added to a flask containing 200cc of dimethylsulfoxide, agitated at 65°C and dissolved the two polymers completely to prepare a polymer blend solution.

To investigate a blood compatibility of the polyurethane-polyvinyl alcohol blend, so prepared, the polymer blend was poured into a glass dish, and dried until the solvent was completely evaporated at an oven at 60°C. The polymer film, so dried, was immersed in distilled water for its complete swelling, followed by stripping off the film out of the dish.

The film was completely washed with ethanol and distilled water, and stored in PBS (phosphate-buffered solution) prior to measurement of the blood compatibility.

### Example 2

10g of polyurethane and 5g of polyhydroxyethylmethyl methacrylate were added to a flask containing 200cc of dimethylformamide, and agitated at 60 °C to prepare a polymer blend solution where the two polymers were completely dissolved.

To investigate a blood compatibility of the polyurethane-polyhydroxyethylmethyl methacrylate blend, the polymer blend was poured into a glass dish, and dried until the solvent was completely evaporated at an oven at 60 °C. The polymer film, so dried, was immersed in distilled water for its complete swelling, followed by stripping off the film out of the dish.

The film was completely washed with ethanol and distilled water, and stored in PBS (phosphate-buffered solution) prior to measurement of the blood compatibility.

### Example 3

10g of polyurethane and 5g of polyacrylic acid were added into a flask containing 200cc of dimethylformamide, and agitated at 60 °C to prepare a polymer blend solution where the two polymers were completely dissolved.

To investigate a blood compatibility of the polyurethane-polyacrylic acid blend, the polymer blend was poured into a glass dish, and dried until the solvent was completely evaporated at an oven at 60 °C. The polymer film, so dried, was immersed in distilled water for its complete swelling, followed by stripping off the film out of the dish.

The film was completely washed with ethanol and distilled water, and stored in PBS (phosphate-buffered solution) prior to measurement of the blood compatibility.

### Example 4

10g of polyurethane and 5g of polyacryl amide were added into a flask containing 200cc of dimethylformamide, and agitated 60°C to prepare a polymer blend solution where the two polymers were completely dissolved.

To investigate a blood compatibility of the polyurethane-polyacry] amide blend, the polymer blend was poured into a glass dish, and dried until the solvent was completely evaporated at an oven at 60 °C. The polymer film, so dried, was immersed in distilled water for its complete swelling, followed by stripping off the film out of the dish.

The film was completely washed with ethanol and distilled water, and stored in PBS (phosphate-buffered solution) prior to measurement of the blood compatibility.

### Example 5

10g of polyurethane and 5g of polyvinyl pyrrolidone were added into a flask containing 200cc of dimethylformamide, and agitated 60°C to prepare a polymer blend solution where the two polymers were completely dissolved.

To investigate a blood compatibility of the polyurethane-polyvinyl pyrrolidone blend, the polymer blend was poured into a glass dish, and dried until the solvent was completely evaporated at an oven at 60°C. The polymer film, so dried, was immersed in distilled water for its complete swelling, followed by stripping off the film out of the dish.

The film was completely washed with ethanol and distilled water, and stored in PBS (phosphate-buffered solution) prior to measurement of the blood compatibility.

### Example 6

10g of polyurethane and 5g of polyethylene oxide were added into a flask containing 200cc of dimethylformamide, and agitated 60°C to prepare a polymer blend solution where the two polymers were completely dissolved.

To investigate a blood compatibility of the polyurethane-polyethylene oxide blend, the polymer blend was poured into a glass dish, and dried until the solvent was completely evaporated at an oven at 60°C. The polymer film, so dried, was immersed in distilled water for its complete swelling, followed by stripping off the film out of the dish.

The film was completely washed with ethanol and distilled water, and stored in PBS (phosphate-buffered solution) prior to measurement of the blood compatibility.

### Example 7: Preparation of polymer membrane

A glass plate was thinly coated with the polyurethane-polyvinyl alcohol blend solution, so prepared in Example 1, and then the dimethylsulfoxide used as was dissolved in ethanol. With the possible phase separation of the polymer blend, a polymer membrane was manufactured.

### Example 8: Preparation of artificial vessel

The glass bar was coated with the polyurethane-polyvinyl alcohol blend solution, so prepared in Example 1. The phase separation of the polymer membrane coated with ethanol was made available and then, the glass bar was dried at room temperature and coated with 10% polyurethane to carry out the phase separation again. The glass bar coated with the polymer was placed in distilled water to remove the solvent, and then, the polymer was pulled out of the glass bar, thus manufacturing an artificial vessel. Also, the artificial vessels were manufactured using the polymer blend solution, so prepared from Examples 2-6.

### Experimental example: Evaluation on antithrombogenesis

To evaluate the antithrombogenetic property of blood-compatibility materials, the following parameters in in-vitro studies are of significant importance in the following methods: changes in the shape of platelet, amounts of adhered platelet and formation of thrombogenesis were investigated.

To observe the changes in the shape of platelet observed through an electron microscope, a platelet-rich plasma was contacted with the mixture of polyurethane, being generally used as a material for artificial organs (e.g., artificial vessel, artificial heart and artificial cardiac valve) and the polymer blends, so prepared in Example 1-6, for 30 minutes.

Further, to observe the amount of platelet adhered, the platelet was separated with Triton x-100 solution, and then the activity of lactate dehydrogenase (LDH) was investigated.

In addition, 1cc of thrombosis was contacted with a polymer film disk in a radius of 16mm to measure the weight of thrombus, so formed. The results were shown in Table 1.

**Table. 1**

| Item | Number of adhered platelet (#/cm²) | Activity of LDH (unit/ ℓ) | Weight of formed thrombus (g) |
|---|---|---|---|
| Polyurethan | 2.6 × 10⁴ | 5.81 | 0.014 |
| Example 1 | 1.0×10⁴ | 1.17 | 0.002 |
| Example 2 | 1.6 × 10⁴ | 2.13 | 0.006 |
| Example 3 | 1.3×10⁴ | 1.78 | 0.004 |
| Example 4 | 1.2×10⁴ | 1.54 | 0.003 |
| Example 5 | 0.9 × 10⁴ | 1.48 | 0.003 |
| Example 6 | 1.4×10⁴ | 1.79 | 0.003 |

From the above Table 1, it was revealed that the polyurethane-hydrophilic polymer blend according to this invention had less amount of adhered platelet compared with a single use of polyurethane.

Also, the LDH activity may be some index representing the amount of adhered platelet. In this respect, the polyurethane-hydrophilic polymer blend had less adhesion to platelet than a single use of polyurethane.

Further, when a thrombus was weighed in the experiment of thrombus coagulation, the polyurethane-hydrophilic polymer blend had smaller weight than a single use of polyurethane.

As a result of confirming the shape of platelet through an electron microscope, a lot of pseudopodium was distributed to platelet adhered to the polyurethane in a wider shape, whereas two blends derived from polyurethane-polyvinyl alcohol and polyurethane-polyhydroxyethylmethyl methacrylate, being adhered to platelet had retained their original shapes of platelet, which remained unchanged.

As mentioned above, it is well understood that the polyurethane-hydrophilic polymer blend of this invention is superior to a polyurethane-based resin in terms of shape of platelet, less amount of platelet adhered, low activity of LDH and less formation of thrombus. With its excellent blood compatibility or anti-thrombogenesis, the polyurethane-hydrophilic polymer blend of this invention may be effectively used in artificial organs and medical devices, which requires antithrombogenic property.

## Claims

1. An anti-thrombogenic polyurethane-hydrophilic polymer blend, wherein the hydrophilic polymer, being solubilized or swelled in water, is homogeneously mixed with the polyurethane-based resin.

2. The anti-thrombogenic polyurethane-hydrophilic polymer blend according to claim 1, wherein the hydrophilic polymer is one or more than two polymers selected from the group consisting of polyvinyl alcohol, polyhydroxyethylmethyl methacrylate, polyacrylic acid, polyacryl amide, polyvinyl pyrrolidone and polyethylene oxide.

3. An artificial organ, characterized in that it comprises the polyurethane-hydrophilic polymer blend as specified in claim 1.

4. The artificial organ according to claim 3, wherein the artificial organ is artificial vessel, artificial kidney, artificial heart, artificial liver, artificial lung or artificial cardiac valve.

5. A medical device, characterized in that it comprises the polyurethane-hydrophilic polymer blend as specified in claim 1.

6. The medical device according to claim 5, wherein the medical device is catheter, sensor or stent.
